# EUROPEAN PATENT APPLICATION

(11) **EP 1 629 771 A1**
(43) Date of publication of application: **01.03.2006**
(21) Application number: 05254470.7
(22) Date of filing: 18.07.2005
(51) Int. Cl.: A61B 5/0408, A61B 5/0478, A61B 5/0492, A61N 1/04

(54) **Electrode apparatus and system**

(30) Priority: 31.08.2004 US 930935
(71) Applicant: Teledyne Technologies Incorporated, West Los Angeles, CA 90064-1021 (US)
(72) Inventor: Grigorov, Ilya L., Los Angeles, CA 90038 (US)
(74) Representative: Finnie, Peter John

(57) **Abstract**

An electrode apparatus and system for obtaining a bio-potential signal. The electrode includes a substrate having a first and second surface. The substrates supports a plurality of discrete conductive structures that are disposed on the first surface of the substrate. The plurality of discrete conductive structures extend outwardly beyond the first surface of the substrate and present an electrically conductive surface to a surface of a stratum comeum layer of skin without penetrating the stratum corneum layer. The system includes the electrode in communication with the electrode for receiving the bio-potential signal from the electrode.

## Description

### Field of the Invention

The present invention is directed generally to electrodes applied to a living body for monitoring bio-potentials. Particularly, the present invention is directed to medical electrodes applied to a skin of a living body. More particularly, the present invention is directed to electrodes that comply with the living body's skin over a surface area thereof.

### Background to the Invention

Proper measurement of human bio-potential signals ("bio-signals") requires a reasonably low active impedance contact between a conducting medium of a living body and the measurement electrode. The external dead layer of the skin, "stratum comeum," is an insulating medium, which, when contacted, presents a high-value reactive impedance with time dependent leakage. This is not acceptable for measurements of bio-signals in the range of a few millivolts and lower with a frequency spectrum of 0 to 200 Hz.

Conventional electrodes formed of Ag/AgCl compounds are widely used for monitoring bio-potential signals. These electrodes may be fabricated using a variety of methods and generally require the use of a conductive gel electrolyte to couple the bio-signals across the living body's skin into the electrodes. Alternatively, to provide adequate electrical coupling between the live skin beneath the insulating layer and the electrodes the "stratum comeum" layer of the skin may be removed prior to attaching the electrode. The common clinical practices of using electrolytic gels and/or removal of the "stratum corneum" layer, however, are cumbersome and do not extend naturally to wearable applications (e.g., applications where the patient wears the electrodes while performing physical activities). A problem that arises in such wearable electrode applications is a phenomenon known as motion artifact where a voltage is induced while the electrode is in motion with respect to the skin and the gel electrolyte. The induced voltage is a noise source comprised of parasitic transients that distort the desired bio- signal waveforms.

Some electrodes are designed to eliminate the motion artifact induced noise. These electrodes include adhesively attachable metal plates, suction-cups, and multipoint flat electrodes deposited on flexible polymeric material, which conforms to the local curvature of the body, and electrodes with needle-like structures that penetrate the "stratum comeum" layer (the insulating layer of skin). The array of the needle-like structures are designed penetrate the "stratum corneum" and provide a direct resistive coupling to a monitoring device attached to the electrode. Therefore, the needle-like structures must be of a predetermined height that is compatible with the thickness of the "stratum comeum" layer. Practically, these needles punch through the "stratum comeum" and directly contact the live tissue there beneath. By penetrating the stratum comeum layer, these electrodes help to eliminate the motion artifact. Furthermore, these electrodes may be used in conjunction with carbonized fiber bands that promote local perspiration and provide a natural substitute for the gel electrolyte. One method of fabricating these electrodes comprises deep anisotropy etching (e.g., deep Micro-Electro-Mechanical System (MEMS)) to form a two dimensional (2-D) array of metal coated Si convex structures or needle-like structures.

Thus, there is a need in the art for a low-impedance, gel-free, motion artifact-free electrode for measuring a subject's bio-signals whether the subject is at rest or in motion relative to the electrode, as well as transposing the bio-signals in applications where subject is physically active.

### Summary of the Invention

In one embodiment, the present invention relates to an electrode for obtaining a bio-signal. The electrode includes a substrate having a first surface and a second surface and a plurality of discrete conductive structures disposed on the first surface of the substrate. In one embodiment, the discrete conductive structures may be metallic, for example. The plurality of discrete conductive structures extend outwardly beyond the first surface of the substrate, and discrete conductive present an electrically conductive surface to a surface of a stratum comeum layer of skin without penetrating the stratum corneum layer.

In another embodiment, the present invention relates to an electrode for obtaining a bio-potential signal. The electrode includes means for supporting comprising a first surface and a second surface and means for contacting a surface of a stratum comeum layer of skin and electrically conducting a current disposed on the first surface of the means for supporting. The means for contacting extend outwardly beyond the first surface of the means for supporting, and the means for contacting present an electrically conductive surface to the surface of the stratum comeum layer of skin without penetrating the stratum comeum layer.

In yet another embodiment, the present invention relates to an electrode system that includes an electrode and a remote device in communication therewith. The electrode includes a substrate having a first surface and a second surface; and a plurality of discrete conductive structures disposed on the first surface of the substrate. The plurality of discrete conductive structures extend outwardly beyond the first surface of the substrate. The plurality of discrete conductive structures present an electrically conductive surface to a surface of a stratum comeum layer of skin without penetrating the stratum comeum layer. The remote device receives the bio-potential signal from the electrode.

### Brief Description of the Drawings

Examples of the present invention will now be described in detail with reference to the accompanying drawings, in which:
Figure 1 is one embodiment of a stud-bump structure electrode applied to a subject's skin;
Figure 2 is one embodiment of a cross-sectional view of the electrode in Figure 1;
Figure 3 is one embodiment of an active electrode;
Figure 4 is one embodiment of a single layer stud-bump structure;
Figure 5 is one embodiment of a multi-layered stacked stud-bump structure;
Figure 6 is one embodiment of a single layer stud-bump structure;
Figure 7 illustrates one embodiment of a multi-layer stud-bump structure electrode applied to a subject's skin; and
Figure 8 is one embodiment of a cross-sectional view of the electrode in Figure 7.

### Detailed Description

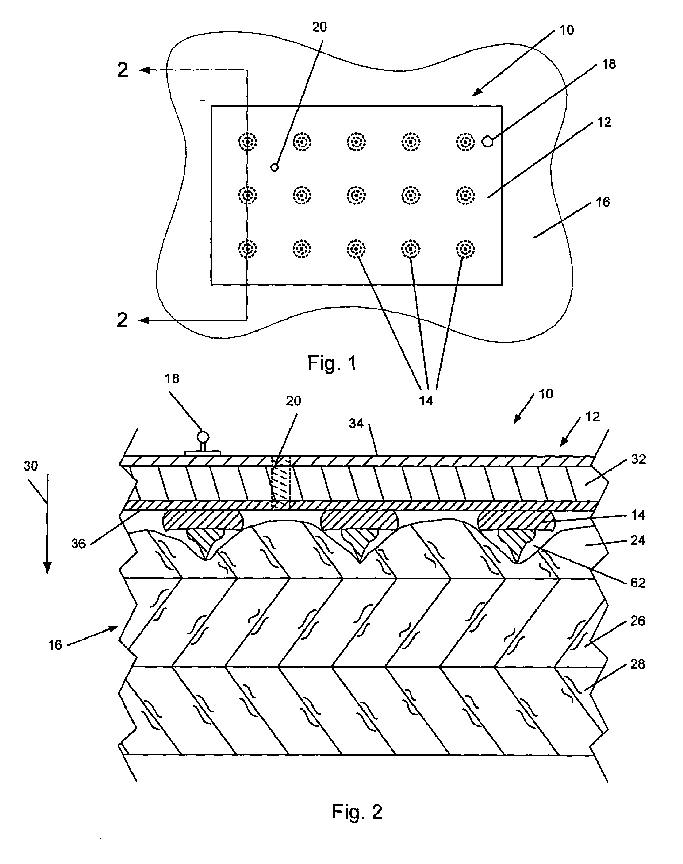

Figure 1 shows one embodiment of an electrode 10 according to the present invention applied to a portion of a subject's skin 16. The electrode 10 comprises, for example, a supporting substrate 12 and a plurality of discrete conductive structures 14 formed thereon. The discrete conductive structures 14 extend outwardly beyond a normal line or surface of the substrate 12. The electrode further includes a terminal 18 for providing an electrical connection to the substrate 12 and/or the plurality of discrete conductive structures 14. The substrate 12 may further include a via 20 for electrically connecting top and bottom portions of the substrate 12 when conductive material is disposed on both sides thereof. The metallic discrete conductive 14 may jut out from the substrate 12 at a sharp angle or may have a more rounded form. At the apex, the discrete conductive structures 14 outwardly extending from the surface of the substrate 12 may comprise a rounded end, a pointy end, a concavity or other similar structural features without departing from the scope of the present invention. The substrate 12 may be rigid, flexible or semi-flexible, for example. In one embodiment, the substrate 12 may be flexible such that it conforms to the contours of the subject's skin 16. In another embodiment, the substrate 12 may be rigid such that the subject's skin 16 conforms to the shape of the substrate 12 or conforms to the structural features on the substrate 12. In yet another embodiment, the substrate 12 may be semi-flexible such that the substrate partially conforms to the subject's skin 16 and the subject's skin 16 partially conforms to the shape of the substrate 12 or to the structural features on the substrate 12 or to the structural features on the substrate 12. In any of these embodiments, however, the purpose of the electrode 12 is to make physical electrical contact with a surface area of the subject's skin 16 and present an electrically conductive surface to the top layer of the skin 16 with as many of the discrete conductive structures 14 as practically possible such that the subject's bio-signals readily may be coupled to the electrode 10. In several embodiments, the electrode 10 may be coupled to the subject's skin 16 without employing external electrolytic compounds between the skin 16 and the electrode 10.

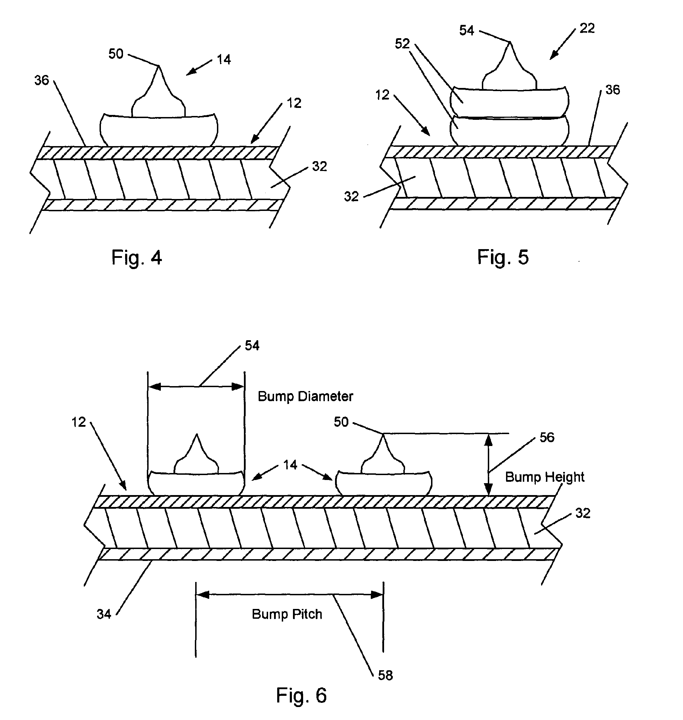

More specifically, in one embodiment, the present invention provides an electrode formed as a two-dimensional array of the discrete conductive structures 14 formed on the substrate 12. Discrete conductive structures 14 that extend outwardly beyond a normal line or surface of the substrate 12 are referred to herein as "stud-bump" structures 14. In general, stud-bump structures are used in the microelectronics industry to form electronic interconnecting devices, for example. A method of forming the stud-bump structures 14 is referred to herein as a "stud-bump process" or simply "stud-bumping," e.g., a specialized process of forming, on the substrate 12, a plurality of discrete conductive structures 14 that extend outwardly beyond a normal line or surface of the substrate 12. As used herein the "stud-bump" and "stud-bumping" nomenclature is merely a concise manner of describing the discrete conductive structures 14 and the process of forming them. This nomenclature, however, is not intended to limit the scope of the invention in any way. The various embodiments of single stud-bump structures 14 and multiple stacked stud-bump structures 22 (Figs. 5, 7, and 8) described herein are suitable for making electrodes 10 having impedances and bio-signal measurement characteristics within practical acceptable ranges. For example, in one embodiment, electrodes 10 formed with stud-bump structures 14, 22 according to the invention demonstrate a low active impedance (e.g., about 500 kOhm) and no motion artifacts when placed on the subject's skin 16.

Figure 2 is a cross sectional view of the electrode 10 applied to the subject's skin 16. The skin 16 is formed of three layers: stratum comeum 24, viable epidermis 26, and dermis 28. The stratum comeum 24 layer is about 10-15 microns thick, and consists of dead tissue. Beneath the stratum comeum 24 layer is the viable epidermis 26 layer. The viable epidermis 26 layer is about 50-100 microns thick and consists of tissue containing living cells. Underneath the viable epidermis 26 layer is the dermis 28 layer and it is comprised of living cells, nerves, and blood vessels.

The stud-bump structures 14 according to one embodiment of the present invention extend from the substrate 12 in an outward direction as indicated by arrow 30. The stud-bump structures 14, however, do not penetrate or pierce any layers of the subject's skin 16. As shown, the stud-bump structures 14 conform to the subject's skin 16 at least at the stratum comeum layer 24. The electrode 10 may be secured to the subject's skin 16 in a variety of ways. In one embodiment, the electrode 10 may be secured to the subject's skin 16 using adhesive tape (not shown). Other suitable adhesives may be applied to a peripheral portion of the electrode 10 to secure it in place.

The stud-bump structures 14 may be formed on a variety of base media substrates made of a plurality of different materials. The stud-bump structures 14 may be fabricated on any suitable rigid (e.g., ceramic, alumina, etc.), flexible or semi-flexible substrate 12, for example. Any substrate media that is suitable for attaching the stud-bump structures 14 thereto may be employed in fabricating the electrode 10. In one embodiment, the stud bump structures 14 may be formed on the substrate 12 that includes a ceramic thick-film base material 32 (e.g., alumina), a first metallic conductive region 34, and a second conductive region 36 formed on the ceramic material 32. The first and second conductive regions 34, 36 each may be formed of one or more metallic layers of Au, Au alloy, Ag, Ag alloy, Al, Al alloy, Cu, Cu alloy, Pd or Pd alloy, for example. The conductive regions 34, 36 may be deposited, sputtered, or printed on the ceramic material 32 using conventional methods, for example. The use of a rigid ceramic material 32 as a base for the substrate 10, however, is not strictly required to implement the electrode 10. For example, the stud-bump structures 14 also may be fabricated on polymer-based flexible circuits with Cu/Ni/Au material plated thereon or Pd/Au material sputtered thereon. Other types of substrates may include, for example, solid metal substrates, non-metallic substrates having a conductive surface or region, and laminate materials (e.g., rigid boards and flex circuits) comprising Au plated Cu conductors formed thereon, and the like. In one embodiment, the non-metallic substrates may include a metallized conductive surface or region thereon, for example.

The conductive regions 34, 36 formed on the ceramic base material 32 may be fabricated in single or multiple layers. In one embodiment, the conductive regions 34, 36 comprise one or more layers of Au, which may be deposited, sputtered, or printed on the ceramic base material 32 using conventional methods. In other embodiments, the conductive regions 34, 36 may be formed of one or more layers of Au alloys, Ag, Ag alloys, Cu, Cu alloys, Al, Al alloys, Pd, Pd alloys, and compounds thereof. The conductive regions 34, 36 provide for low-activation energy formation of the intermetallics with the Au, Au alloys, Ag, Ag alloys, Cu, Cu alloys, Al, Al alloys, Pd, and Pd alloys, and compounds thereof. Although the most commonly used metals for forming the conductive regions 34, 36 are Au, Ag, Cu, Al, Pd, and alloys and compounds thereof, other metals, their alloys, and their compounds may be used without departing from the scope of the invention.

In one embodiment the two-dimensional array of stud-bump structures 14 may be fabricated on an alumina substrate 32 with an Au thick film conductive region 36 on one side and an Ag thick film conductive region 34 on the other, for example. The two conductive regions 34, 36 may be electrically connected through one or more vias 20, for example. The stud-bump structures 14 are formed on the Au conductive region 36 using Au/1% Pd wire, for example. The Ag conductive region 34 may be used, for example, for attaching the terminal 18 or copper leads directly to the electrode 10 using soldering techniques. In contrast with conventional stud-bump fabrication methods, the two-dimensional array of stud-bump structures 14 may be formed of a single suitable material and/or a single suitable metallic material and does not require additional deposition of metal thereon.

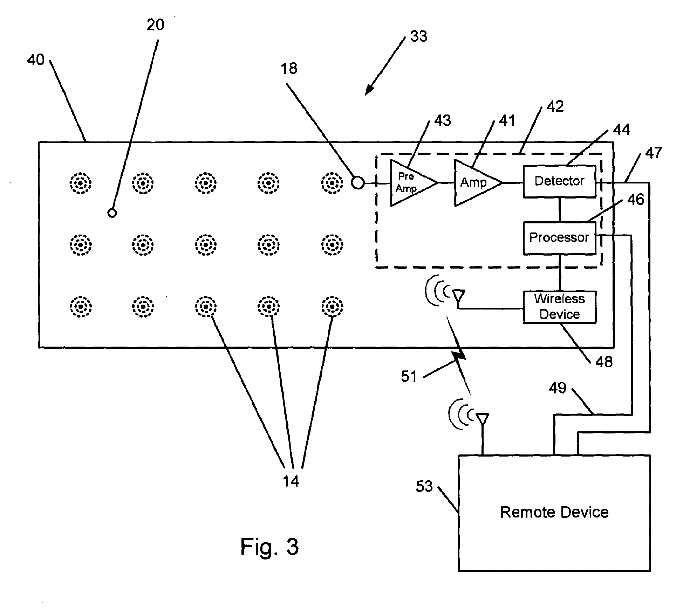

Figure 3 shows an active electrode 38 comprising a substrate 40 that includes an electronic signal conditioning circuit 42 thereon. In one embodiment, the active electrode 38 may include one or more amplifiers 41 and/or pre-amplifiers 43. In other embodiments, the one or more signal conditioning circuit 42 may be located remotely from the electrode 38 and connected thereto by way of an electrical cable attached to the terminal 18 or soldered directly on the Ag conductive region 34, for example. The remote configuration, may add unwanted noise signals to the bio-potential signals of interest depending on the length of the cable. Because, the stud-bump manufacturing process also is compatible with other electronic assembly technologies, such as hybrid and surface mount technology, the active electrode 38 also may include any number of electronic circuits, such as, for example, integrated detector circuits 44, processing circuits 46 for processing signals or data, and/or a communication device, such as for example the wireless device 48. The communication device may include, for example, a wireless transmission circuit, wireless receiver circuit or a wireless transceiver circuit which would eliminate the need for electrical cables. The communication device also may include wireless transmission, receiver, and transceiver circuits, for example. Those skilled in the art will appreciate that detection of the bio-signals may be performed in accordance with known art apparatuses, systems, and methods. The bio-signals may be provided to a remote device 53. In one embodiment, the bio-signals may be transferred to the remote device 53 via a wired analog link 47 or a wired digital link 49. In another embodiment, the bio-signals may be transferred the remote device 53 via a wireless link 51. In one embodiment, the remote device 53 may be a terminal (e.g., video monitor, electrocardiogram (ECG) monitor, and the like) and in another embodiment, the remote device 53 may be a recorder (e.g., tape, ECG tape, and the like).

Figure 4 shows an embodiments of the stud-bump structure 14 using a stud-bumping process. At the apex 50, the stud-bump structure 14 may comprise a rounded end, a pointy end, a concavity or other similar structural features without departing from the scope of the present invention. In one stud-bumping process, a metallic wire is used to form the stud-bump structure 14 over a metal-coated conductive region 36 of the substrate 12. The metallic wire may be Au, Al, Cu, Pd, alloys thereof, compounds thereof, and/or any other suitable metallic material, for example. In other embodiments, the single stud-bump structure 14 may be formed using one or more of a plurality of materials including, but not limited to, Mg, Al, Ti, Mn, Fe, Co, Ni, Cu, Pt, Pd, In, Sn, W, Au, Ag, Ir, Pb, Pd, Tb, non-metals Si, P, S, Cl, Ge, C, N, O, alloys thereof, and compounds thereof or any other suitable metals, alloys or compounds thereof without limiting or departing from the scope of the invention.

Figure 5 shows an embodiment of a multi-layered stacked stud-bump structure 22 formed as a plurality of discrete multi-layered or stacked elements 52 formed one on top of the other. Like the single layer stud-bump structures 14 described previously, the multi-layered stud-bump structures 22 may jut out from the substrate 12 at a sharp angle or may have a more rounded form. At the apex 54, the multi-layered stud-bump structures 22 may comprise a rounded end, a pointy end, a concavity or other similar structural features without departing from the scope of the present invention.

The single and multi-layered stud-bump structures 14, 22 may be fabricated using conventional stud-bumping processes, for example. These processes may include, for example, conventional semiconductor wire-bonding techniques and use conventional semiconductor wire-bonding equipment. Stud-bump structures 14, 22 are truncated elements of thermosonic ball bonds fabricated with a thermosonic ball wire-bonding machine. Thermosonic ball bonding is widely used in the semiconductor industry and the process is readily understandable to those skilled in the art. Stud-bump technology is used for attaching flip-chip semiconductor dies to a substrate and, for example, for interconnecting two or more passive components to a particular region of the substrate. In general, flip-chip bonding is a method of attaching a die comprising Au bumps formed undemeath the die to bonding pads on the substrate by applying ultrasonic vibration, heat, and force to the die. The Au bumps beneath the die are forced against the opposing bonding pads (e.g., conductive regions formed on the substrate), and a second metallic bond is formed where the bump comes in contact with the die package metallization. The top and bottom connection of the stud-bump structures 14, 22 form similar connections and constitute a true metal-to-metal connection.

Referring back to Figure 4, for example, in one embodiment, the single layer stud-bump structures 14 may be formed using a wire-bonding process using a conductive wire (e.g., Au, Al, Cu, Pd, alloys thereof, compounds thereof or any other suitable metals, alloys or compounds, etc.) fed through a ceramic capillary. The ceramic capillary has a first end adapted for receiving the wire and a second end having a specifically machined shape (e.g., a chamfered end). The free end of the wire protrudes from the second end of the capillary and the first end of the capillary is attached to an ultrasonic transducer. Electric discharge is applied to the first end of the capillary and a spark from the discharge forms a uniform metallic ball on the free end of the wire protruding from the second end of the capillary. The spark melts the free end of the wire to form the uniform metallic ball. Once the ball is formed on the conductive region 36 the capillary presses the ball against the conductive pad. Ultrasonic energy in the range of 60-120 kHz is applied to the ball through the capillary to form a metal-to-metal weld connection or metallic bond between the ball material and the conductive region 36 of the substrate 12. Different processes may accommodate various ranges of ultrasonic energy. In one embodiment, ultrasonic energy in the range of 40-200 kHz may be applied without departing from the scope of the invention. The formation of the bond is accompanied by the deformation of the ball squeezed between the chamfered end of the capillary and the pad.

In conventional wire-bonding applications, once the original ball is bonded to the conductive region 36 more wire is fed through the capillary as it moves to a second position on the substrate 12 to form a second bond on the substrate. In conventional wire-bonding, the ceramic capillary loops the wire while it is still attached to the welded ball to the second bonding pad. In a stud-bumping process, however, no looping action is required. Rather, when forming single layer stud-bump structures 14, the movement of the wire is restricted as the capillary pulls away from the bonded ball. Once the capillary pulls away from the welded ball, the wire-bonding system clamps the wire above the capillary and yanks on it to break the wire away from the ball. The wire breaks at the neck right above the ball in what is referred to as the "heat affected zone." This is the wire's most fragile section and is located just above the ball. The breaking process leaves a short length of wire called the tail protruding from the ball and sticking upwards above the surface of the ball. The resulting structure is the single layer stud-bump structure 14.

With reference now to Figure 6, the single layer stud-bump structure 14 may be characterized by several parameters, such as, for example, a stud-bump diameter 54, the maximum lateral dimension of the deformed ball, and the stud-bump height 56, the distance from the surface of the bonding conductive region 36 to the apex 50 of the single layer stud-bump structure 14. The dimensions of the diameter 54 and the height 56 of the single layer stud-bump structure 14 may be process controlled. To form an optimal metallurgical bond (e.g., metal-to-metal connection) between the single layer stud-bump structure 14 and the conductive region 36, a stud-bump diameter 54 of 2.5-3.5 times the wire diameter may be preferred. Tight process control can accomplish consistent bonding with a stud-bump diameter 54 of about two-times the wire diameter. The height 56 of the single layer stud-bump structure 14 is mostly defined by the size of the original undistorted deform-free ball and the stud-bump diameter 54. The length of the protruding tail (e.g., the remaining portion of the wire above the ball after breaking in the heat-affected zone) is generally consistent. The tail may be eliminated by shearing it right above the ball or elongated by using external means for cutting the wire. The length of the tail also may be kept near the desired dimension by process control, machine-control by a secondary electrical discharge or by mechanically cutting the tail to the desired dimension. These processes will change the length of the tail in proportion to the height of the deformed ball.

In one embodiment, the single layer stud-bump structure 14 may be formed with a diameter 54 ranging from 25 to 150 microns, for example, using conventional wire. In another embodiment, the stud-bump structures 14 may be formed with diameters 54 in the range of 50 to 100 microns, for example. In one embodiment, the single layer stud-bump structure 14 with stud-bump diameters 54 of 60-65 microns and 48-50 microns may be formed using conventional 25-micron wire (*i*.*e*., 25 microns in diameter). The smallest diameter wire currently manufactured for wire-bonding applications is 17.5 microns. With this wire diameter, stud-bump diameters 54 of 35 microns may be attained. The ball height 56 for a 60-65 micron diameter 54 single layer stud-bump structure 14 is about 20 microns. For a 50 micron stud-bump diameter 54, the height 56 may be reduced to 10-12 microns, for example. In one embodiment, the stud-bump structures 14 may be formed with a height 56 range of 10 to 250 microns. In another embodiment, the stud-bump structures 14 may be formed with a height 56 range of 35 to 120 microns.

The size of the ball or the ball's total volume is dependent on the energy output of the spark. Thus the size of the ball may be controlled by controlling by the power of the electrical discharge. In one embodiment, the single layer stud-bump structures 14 may be formed with a diameter 54 of 60 microns and a height 56 of 60 microns, wherein the height 56 includes the height of the tail of 35 microns. The pitch 58 is the distance between any two stud-bump structures 14, and is programmable with micron-level accuracy. In one embodiment the pitch 58 may be maintained at approximately 150 microns. If an increased stud-bump height 56 is desired, two or more stud-bump structures may be stacked or layered and ultrasonically welded one on top of the other. A two-layered stud-bump structure 22 is shown in Figure 5. The shape of the multi-layered stud-bump structure 22 may not be as precise as the shape of the initial stud-bump formed on the conductive region 36 because the lateral dimensions may change while ultrasonically welding the additional stud-bump on top of an existing stud-bump. The height of the two-layered stud-bump 22 is approximately 90 microns. Three- or four-high stacked multi-layer stud-bump structures may be fabricated, but as the number of layers increases above two, they become less practical to implement. Another technique for increasing the height of the stud-bump includes cutting the wire above the ball using electrical discharge or mechanical means. These external cutting techniques leave a long, soft tail extending above the stud-bump and have a diameter similar to that of the wire.

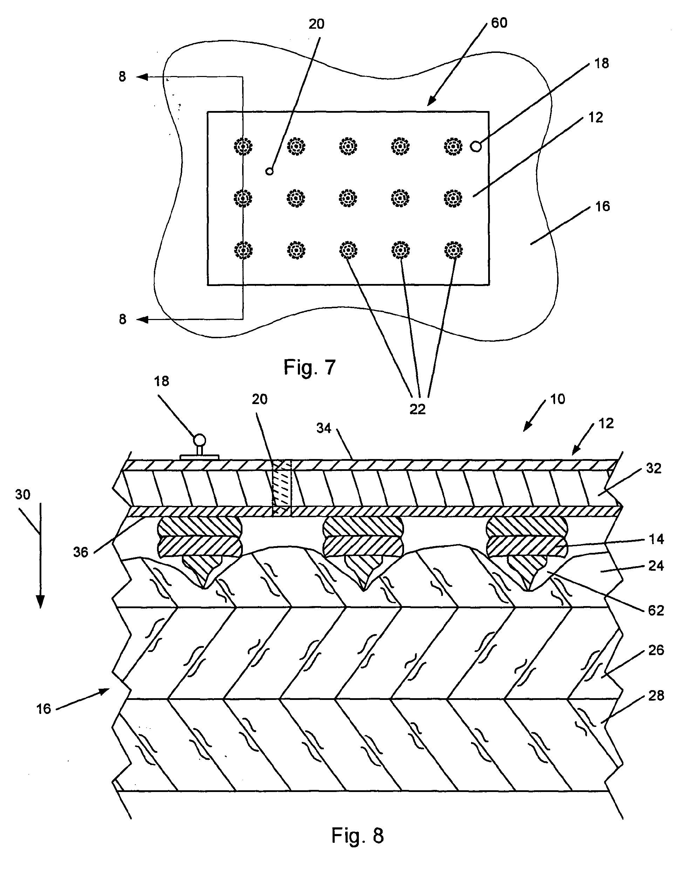

Figures 7 and 8 show an electrode 60 applied to the subject's skin 16. The electrode 60 includes the substrate 10 with an array of the multi-layer stud-bump structures 22 formed thereon.

The functionality of conventional dry electrodes comprising two-dimensional arrays of vertically projecting conductive structures is based on the conductive structures penetrating the skin 16 through the "stratum comeum" layer 24 to the viable layer 36. Thus, these conventional structures are hard, sharp, and have a streamlined shape capable of easily penetrating the subject's skin 16 beyond the "stratum comeum" layer 24. The stud-bump structures 14, 22 (e.g., single stud-bump and multi-layered stud-bump structures) according to various embodiments of the present invention, however, do not have long, sharp protrusions, and may be generally formed of soft materials (e.g., as described above the stud-bumps may be formed of Mg, Al, Ti, Mn, Fe, Co, Ni, Cu, Pt, Pd, In, Sn, W, Au, Ag, Ir, Pb, Pd, Tb, non-metals Si, P, S, Cl, Ge, C, N, O, alloys thereof, and compounds thereof or any other suitable metals, alloys or compounds thereof, and the like). Applying the electrode 10, 38, 60, comprising an array of the stud-bump structures 14, 22 according to the various embodiments of the present invention, to dry skin produces an extremely high impedance contact because the stud-bump structures 14, 22 do not penetrate the "stratum comeum" layer 24 of the skin 16. Thus, a different mechanism is responsible for the performance of the electrode 10, 38, 60 according to the various embodiments of the present invention.

The stud-bumps structures 14, 22 according to various embodiments of the present invention neither puncture nor penetrate the subject's skin 16 below the "stratum corneum" layer 24. Rather, the skin 16 deforms and complies to the contours and curvatures of the stud-bump structures 14, 22 regardless of whether the substrate 12, 40 is rigid, flexible or semi-flexible. As a result, the total effective contact area between the skin 16 and the electrode 10, 38, 60 is substantially greater than conventional pierce-the-skin type electrodes. The curvature of the skin 16 where the electrode 10, 38, 60 is applied provides a "trapping" where moisture containing natural bodily ionics form a conducting electrolyte in a region 62 (Fig. 2) between the "stratum comeum" 24 layer of the skin 16 and the electrode 10, 38, 60. Furthermore, the curvature of the skin 16 below the electrode 10, 38 prevents it from easily sliding against the skin 16 and thus eliminates any motion artifact. Moreover, electrodes 10, 38 with flexible substrates such as polymer films provide air-restricting qualities that may induce additional local perspiration, which provides a positive effect in the overall function of the electrode 10, 38, 60, as described below. In one embodiment, the region 62 between the electrode 10, 38, 60 substrate 12, 40 and the stud-bump structure 14, 22 traps a combination of bodily ionics and non-bodily non-ionic liquids that form a conducting electrolyte in the region 62. In another embodiment, the region 62 traps a combination of bodily ionics and non-bodily ionic liquids that form a conducting electrolyte in the region 62. In yet another embodiment, the region 62 traps a combination of bodily ionics and non-bodily ionics that form a conducting electrolyte in the region 62.

At least two parameters of the two-dimensional array of stud-bump structures 14, 22 define the operating region of the electrode 10, 38, 60 that relies on the electrolyte trapping action. First, the pitch 58 (e.g., the distance between any two stud-bump structures 14 in a uniform array of stud-bumps) of the array should allow for the skin 16 to comply with the contours of the array of stud-bump structures 14, 22 formed on the substrate 12, 40. A suitable pitch 58 may be obtained by approximately programming the wire-bonder, for example. Those skilled in the art will appreciate that the minimum practical value of the pitch 58 is the stud-bump diameter 54. Second, an appropriate aspect ratio of the stud-bump height 56 to the pitch 58 is required. If this ratio is too small, the electrode 10, 38,60 may function as a flat piece of metal and would fail to provide the trapping region 62.

In one embodiment, the pitch 58 to diameter 54 ratio (e.g., pitch/diameter) is in the range of approximately 1 to 100. In another embodiment, the pitch 58 to diameter 54 ratio (e.g., pitch/diameter) is in the range of approximately 1 to 10. Furthermore, in one embodiment, the stud-bump structures 14 may be formed with a height 56 to pitch 58 ratio (e.g., height/pitch) in the range of approximately 0.01 to 10. In another embodiment, the stud-bump structures 14 may be formed with a height 56 to pitch 58 ratio (e.g., height/pitch) in the range of approximately 0.1 to 5. Another parameter defining the performance of the electrode 10, 38, is the total number of stud-bump structures 14, 22 located on the substrate 12, 40 (density) as a result of the probabilistic nature of the electrolyte "trapping" action. In certain embodiments, the number of stud-bump structures 14, 22 may be any number capable of providing the electrical conductivity desired for a specific application. In certain applications, the number of the stud-bump structures 14, 22 may range, for example, from 10-10,000 single-layer or multi-layer stud-bump structures 14, 22 on a suitable substrate 12, 40.

The test results indicate the suitability of the described stud-bump structures 14, 22 for use as bio-signal electrodes 10, 38, 60. Although the height 56 of the structures (60-90 microns) may be higher than the thickness of the "stratum comeum" layer 24 (10-50 microns), the stud-bump structures 14, 22 do not penetrate the "stratum comeum" layer 24 of the subject's skin 16. Rather, the conductivity mechanism is largely due to the entrapment of moisture in the highly extended active area of the electrode 10, 38, 60 in the region 62 (Fig. 2) between the substrate 10, 38 and the subject's skin 16. To prevent penetration of the subject's skin 16, the height 56, the height 56-to-pitch 58 ratio, and the hardness of the of the stud-bump structures 14, 22 is monitored and controlled. The height 56, the height 56-to-pitch 58 ratio may be controllable within a standard process. The hardness may be controlled by the choice of materials and by preventing annealing during fabrication. In general, however, even non-optimized electrodes 10, 38, 60 according to embodiments of the present invention exhibit adequate electrical characteristics and no motion artifact.

The main benefits of the stud-bumping process according to the various embodiments of the present invention described above versus a deep MEMS process to fabricate needles are: (1) ease and cost of manufacturing; (2) compatibility with variety of materials to improve usability; (3) easy integration of the resulting structures with read-out; (4) signal processing; (5) and transmission electronics.

With reference to Table 1 below, several impedance measurements were made between pairs of similar 65 micron electrodes 10 and 90 micron electrodes 10 applied to a subject's skin 16. The 65 micron electrodes 10 were fabricated with stud-bump structures having a height 56 of 65 microns. The skin 16 condition parameter was prepared according to the following procedure:
Dry = no skin preparation, electrodes applied to the skin with light pressure;
Moist = a small amount of water applied to the skin with excess removed by paper towel, electrodes applied to the skin with light pressure; and
Wet = a drop of water placed on each electrode surface and applied wet, electrodes applied to the skin with light pressure.

**Table 1**

| Dry Electrode DC Impedance Measurements | | |
|---|---|---|
| Electrode type | Skin condition | Impedance (Ohms) |
| 65 micron - 65 micron | Dry | >15 M |
| 65 micron - 65 micron | Moist | 2.3 M |
| 65 micron - 65 micron | Wet | 540 k |
| 90 micron-90 micron | Dry | >15 M |
| 90 micron-90 micron | Moist | 2.2 M |
| 90 micron-90 micron | Wet | 520 k |

The electrode 10 to skin 16 interaction was measured with the 65 micron stud-bump structures 14 and the 90 micron stud-bump structures 14 electrodes 10. A lower impedance signal path for physiological recording was obtained without the stud-bump structures 14 superficially penetrating the "stratum corneum" layer 24 of the skin 16. Neither the 65 micron and 90 micron electrodes 10 penetrated the "stratum comeum" layer 24. Upon close examination of the skin 16 after applying light pressure to the electrodes 10, the 65 micron electrode 10 left no visible mark on the skin 16, whereas the 90 micron electrode left a very faint square impression. The 90 micron electrode 10 creates only a small indentation without any individual stud-bump structure 14 actually penetrating the "stratum comeum" layer 24.

The electrode's 10 impedance also was measured. As shown in Table 1, the 65 micron and the 90 micron electrodes 10 show essentially the same skin 16 impedance characteristics.

The physiological bio-signal quality for both the 65 micron and the 90 micron electrodes 10 is good. In one embodiment, the 90 micron electrode 10 in the moist and wet skin conditions provides a bio-signal that is more stable than using flat Au or Ag electrodes. This is because the stud-bump structures 14 holds a small amount of moisture by surface tension and thus provides a stable conductive media that is resistant to movement artifacts.

## Claims

1. An electrode for obtaining a bio-potential signal, comprising:
a substrate having a first surface and a second surface; and
a plurality of discrete conductive structures disposed on the first surface of the substrate, wherein the plurality of discrete conductive structures extend outwardly beyond the first surface of the substrate, and wherein the plurality of discrete conductive structures present an electrically conductive surface to a surface of a stratum corneum layer of skin without penetrating the stratum comeum layer.

2. The electrode of claim 1, further comprising a first metallic conductive region on the first surface of the substrate.

3. The electrode of claim 2, wherein the plurality of discrete conductive structures are formed on the first metallic conductive region on the first surface of the substrate.

4. The electrode of claim 2, further comprising a second metallic conductive region on the second surface of the substrate.

5. The electrode of claim 4, wherein the first and second metallic conductive regions are in electrical communication.

6. The electrode of claim 5, further comprising a terminal in electrical communication with the second metallic conductive region.

7. The electrode of claim 1, wherein the plurality of discrete conductive structures comprises single layer stud-bump structures.

8. The electrode of claim 1, wherein the plurality of discrete conductive structures comprises multi-layer stud-bump structures.

9. The electrode of claim 1, wherein the plurality of discrete conductive structures have a diameter that ranges from 25 microns to 150 microns.

10. The electrode of claim 1, wherein the plurality of discrete conductive structures have a diameter that ranges from 50 microns to 100 microns.

11. The electrode of claim 1, wherein the plurality of discrete conductive structures have a height that ranges from 10 microns to 250 microns.

12. The electrode of claim 1, wherein the plurality of discrete conductive structures have a height that ranges from 35 microns to 120 microns.

13. The electrode of claim 1, wherein the plurality of discrete conductive structures have a pitch to diameter ratio in the range of approximately 1 to 100.

14. The electrode of claim 1, wherein the plurality of discrete conductive structures have a pitch to diameter ratio in the range of approximately 1 to 10.

15. The electrode of claim 1, wherein the plurality of discrete conductive structures have a height to pitch ratio in the range of approximately 0.01 to 10.

16. The electrode of claim 1, wherein the plurality of discrete conductive structures have a height to pitch ratio in the range of approximately 0.1 to 5.

17. The electrode of claim 1, wherein the plurality of discrete conductive structures comprises 10-10,000 discrete conductive structures.

18. The electrode of claim 1, further comprising an amplifier in communication with the plurality of discrete conductive structures.

19. The electrode of claim 18, further comprising a signal detector in communication with the amplifier.

20. The electrode of claim 18, further comprising a processor in communication with the amplifier.

21. The electrode of claim 18, further comprising a wireless device in communication with the amplifier.

22. The electrode of claim 1, wherein the plurality of discrete conductive structures are formed of any one of Mg, Al, Ti, Mn, Fe, Co, Ni, Cu, Pt, Pd, In, Sn, W, Au, Ag, Ir, Pb, Pd, Tb, non-metals Si, P, S, Cl, Ge, C, N, O, alloys thereof, and compounds thereof..

23. The electrode of claim 1, wherein the substrate and the plurality of discrete conductive structures conform to a top layer of the stratum comeum and forms a region for trapping bodily ionics that form a conducting electrolyte in the region.

24. The electrode of claim 1, wherein the substrate and the plurality of discrete conductive structures conform to a top layer of the stratum comeum and forms a region for trapping a combination of bodily ionics and non-bodily non-ionic liquids that form a conducting electrolyte in the region.

25. The electrode of claim 1, wherein the substrate and the plurality of discrete conductive structures conform to a top layer of the stratum comeum and forms a region for trapping a combination of bodily ionics and non-bodily ionic liquids that form a conducting electrolyte in the region.

26. The electrode of claim 1, wherein the substrate and the plurality of discrete conductive structures conform to a top layer of the stratum comeum and forms a region for trapping a combination of bodily ionics and non-bodily ionics that form a conducting electrolyte in the region.

27. An electrode for obtaining a bio-potential signal, comprising:
means for supporting comprising a first surface and a second surface; and means for contacting a surface of a stratum comeum layer of skin and electrically conducting a current disposed on the first surface of the means for supporting, wherein the means for contacting extend outwardly beyond the first surface of the means for supporting, and wherein the means for contacting present an electrically conductive surface to the surface of the stratum corneum layer of skin without penetrating the stratum corneum layer.

28. An electrode system, comprising:
an electrode for obtaining a bio-potential signal, the electrode comprising:
a substrate having a first surface and a second surface; and
a plurality of discrete conductive structures disposed on the first surface of the substrate, wherein the plurality of discrete conductive structures extend outwardly beyond the first surface of the substrate, and wherein the plurality of discrete conductive structures present an electrically conductive surface to a surface of a stratum corneum layer of skin without penetrating the stratum comeum layer; and
a remote device in communication with the electrode for receiving the bio-potential signal from the electrode.

29. The system of claim 28, wherein the electrode further comprises a first metallic conductive region on the first surface of the substrate.

30. The system of claim 29, wherein the plurality of discrete conductive structures are formed on the first metallic conductive region on the first surface of the substrate.

31. The system of claim 29, wherein the electrode further comprises a second metallic conductive region on the second surface of the substrate.

32. The system of claim 31, wherein the first and second metallic conductive regions are in electrical communication.

33. The system of claim 32, wherein the electrode further comprises a terminal in electrical communication with the second metallic conductive region.

34. The system of claim 28, wherein the plurality of discrete conductive structures comprises single layer stud-bump structures.

35. The system of claim 28, wherein the plurality of discrete conductive structures comprises multi-layer stud-bump structures.

36. The system of claim 28, wherein the plurality of discrete conductive structures have a diameter that ranges from 25 microns to 150 microns.

37. The system of claim 28, wherein the plurality of discrete conductive structures have a diameter that ranges from 50 microns to 100 microns.

38. The system of claim 28, wherein the plurality of discrete conductive structures have a height that ranges from 10 microns to 250 microns.

39. The system of claim 28, wherein the plurality of discrete conductive structures have a height that ranges from 35 microns to 120 microns.

40. The system of claim 28, wherein the plurality of discrete conductive structures have a pitch to diameter ratio in the range of approximately 1 to 100.

41. The system of claim 28, wherein the plurality of discrete conductive structures have a pitch to diameter ratio in the range of approximately 1 to 10.

42. The system of claim 28, wherein the plurality of discrete conductive structures have a height to pitch ratio in the range of approximately 0.01 to 10.

43. The system of claim 28, wherein the plurality of discrete conductive structures have a height to pitch ratio in the range of approximately 0.1 to 5.

44. The system of claim 28, wherein the plurality of discrete conductive structures comprises 10-10,000 discrete conductive structures.

45. The system of claim 28, wherein the electrode further comprises an amplifier in communication with the plurality of discrete conductive structures.

46. The system of claim 45, wherein the electrode further comprises a signal detector in communication with the amplifier.

47. The system of claim 45, wherein the electrode further comprises a processor in communication with the amplifier.

48. The system of claim 45, wherein the electrode further comprises a wireless device in communication with the amplifier.

49. The system of claim 28, wherein the plurality of discrete conductive structures are formed of any one of Mg, Al, Ti, Mn, Fe, Co, Ni, Cu, Pt, Pd, In, Sn, W, Au, Ag, Ir, Pb, Pd, Tb, non-metals Si, P, S, Cl, Ge, C, N, O, alloys thereof, and compounds thereof..

50. The system of claim 28, wherein the substrate and the plurality of discrete conductive structures conform to a top layer of the stratum comeum and forms a region for trapping bodily ionics that form a conducting electrolyte in the region.

51. The system of claim 28, wherein the substrate and the plurality of discrete conductive structures conform to a top layer of the stratum comeum and forms a region for trapping a combination of bodily ionics and non-bodily non-ionic liquids that form a conducting electrolyte in the region.

52. The system of claim 28, wherein the substrate and the plurality of discrete conductive structures conform to a top layer of the stratum comeum and forms a region for trapping a combination of bodily ionics and non-bodily ionic liquids that form a conducting electrolyte in the region.

53. The system of claim 28, wherein the substrate and the plurality of discrete conductive structures conform to a top layer of the stratum comeum and forms a region for trapping a combination of bodily ionics and non-bodily ionics that form a conducting electrolyte in the region.

54. The system of claim 28, wherein the remote device is a terminal.

55. The system of claim 54, wherein the terminal is a video monitor.

56. The system of claim 54, wherein the terminal is an ECG monitor.

57. The system of claim 28, wherein the remote device is a recorder.

58. The system of claim 57, wherein the recorder is a tape.

59. The system of claim 58, wherein the tape is an ECG tape.
